# EUROPEAN PATENT APPLICATION

(11) **EP 3 620 453 A1**
(43) Date of publication of application: **11.03.2020**
(21) Application number: 18193198.1
(22) Date of filing: 07.09.2018
(51) Int. Cl.: C07D 215/233

(54) **NEW CRYSTAL FORM OF LENVATINIB**

(71) Applicant: Indena S.p.A., 20139 Milano (IT)
(72) Inventor: SENALDI, Luca, 20139 MILANO (IT); ALLEGRINI, Pietro, 20139 MILANO (IT); CICERI, Daniele, 20139 MILANO (IT); RICOTTI, Maurizio, 20139 MILANO (IT)
(74) Representative: Bianchetti Bracco Minoja S.r.l.

(57) **Abstract**

The present invention provides a novel crystalline form of Lenvatinib mesylate, 4-[3-Chloro-4-(N'-cyclopropylureido)phenoxy]-7-methoxyquinoline-6-carboxamide methanesulfonate, a process for its preparation, AND pharmaceutical compositions comprising the novel polymorphic form of Lenvatinib mesylate as active ingredient, in admixture with pharmaceutical excipients.

## Description

The present invention provides a novel crystalline form of Lenvatinib mesylate, 4-[3-Chloro-4-(N'-cyclopropylureido)phenoxy]-7-methoxyquinoline-6-carboxamide methanesulfonate, as well as a process for its preparation.

### BACKGROUND OF THE INVENTION

Lenvatinib mesylate, 4-[3-Chloro-4-(N'-cycloprpylureido)phenoxy]-7-methoxyquinoline-6-carboxamide methanesulfonate, of formula (I) 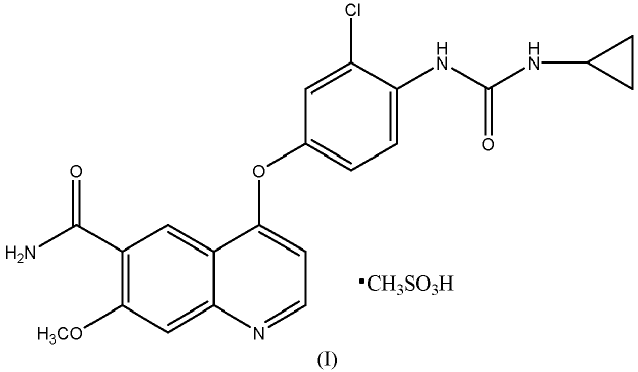 is used to treat differentiated thyroid carcinoma not responding to treatment with radioactive iodine. Lenvatinib is also active against advanced renal cell carcinoma.

Lenvatinib mesylate is known to have a complex polymorphic behavior. Crystalline forms, named A, B, C, F, I and DMSO solvate are described in US 2007/0078159 and other solid forms, named DMSO-1, DMSO-2, ACA-1, ACA-1-HT dry, CHF-1, FOA-1 and H2O-1 are disclosed in WO2018054792. Another solid form is described in WO 2016/184436 and the amorphous form in EP 1 894 918.

Lenvatinib is administered in capsules at a dose of 4 mg or 10 mg. Therefore, there is a strong interest in making available new crystalline forms with low particle size, suitable for the formulation of said capsules.

### DESCRIPTION OF THE INVENTION

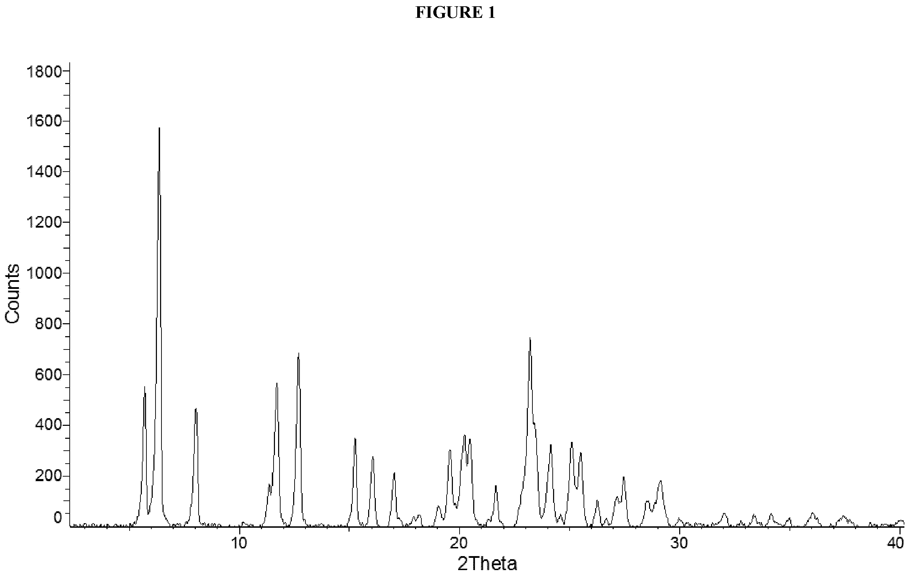

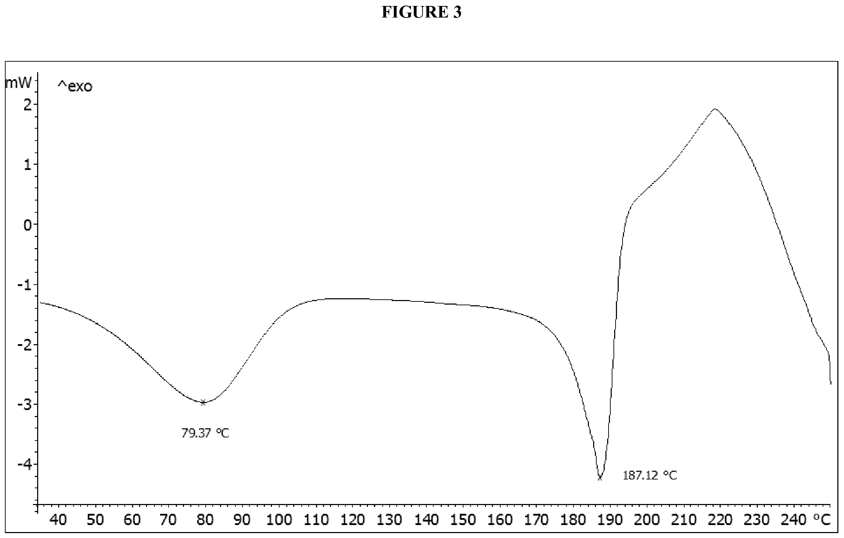

The invention concerns a new polymorph of Lenvatinib mesylate, characterized by specific XRPD, FTIR and DSC data, as illustrated in Figures 1 - 3; the invention further relates to a process for the preparation of the polymorph. The new polymorph is characterized by a narrow homogeneous particle size, with average dimensions of 1-2 microns.

The new crystal form can be prepared by micronization of the Lenvatinib mesylate ACA-1-HT-dry form disclosed in WO2018054792. The Lenvatinib mesylate ACA-1-HT-dry form is an anhydrous crystalline form, which is obtained by heating a sample of the ACA-1 form in an oven at a temperature from 80 to 160°C and a pressure from 1 to 10⁻²atm. The ACA-1 form is a crystalline solvate which is obtained by dissolving Lenvatinib mesylate in acetic acid, at a temperature ranging from room temperature to the boiling point of the solvent. The solution, after filtration and evaporation at 40-80°C at room pressure, yields the crystalline acetate solvate.

The micronization can be carried out by known methods, preferably by means of a jet mill fed with a pressurized inert gas, typically nitrogen. Typically, Lenvatinib mesylate ACA-1-HT dry form is fed portion-wise into a jet mill under specific alimentation and micronization pressures. The feed rate ranges typically from 1 g/min to 50 g/min per unit volume (L) of the micronization chamber preferably 10 g/min. , within a time ranging from 5 to 15 min. Typically, the micronization pressure is set between 4 and 10 bar preferably 6 bar, while the feed pressure is set to 1 bar below the micronization pressure. When the feeding of the ACA-1-HT dry form is complete, the feed pressure is lowered to 0 bar, while the micronization pressure is kept at the initially set value. The micronization time preferably ranges from 5 to 20 min, and is more preferably of 10 minutes. When micronization is complete, the resulting Lenvatinib mesylate solid form of the invention is recovered.

The invention also provides pharmaceutical compositions comprising the novel polymorphic form of Lenvatinib mesylate as active ingredient, in admixture with pharmaceutical excipients.

Details of the preparation and the characterization of the new form are reported in the following example.

### Description of the figures

Figure 1: XRPD spectrum of new polymorph of Lenvatinib
Figure 2: FTIR spectrum of new polymorph of Lenvatinib
Figure 3: DSC analysis of new polymorph of Lenvatinib

### Example

10.0 g of Lenvatinib mesylate, ACA-1-HT dry form were micronized in a MICRONET M100 (Nuova Guseo srl). The sample was introduced portion-wise in 10' with:
- alimentation pressure = 5 bar
- micronization pressure = 6 bar.

After the addition was complete, the feed pressure was decreased to 0 bar, while micronization pressure was maintained at 6 bar for 10'. The solid was recovered (7.3 g) and characterized by XRPD, FTIR and DSC.

The diffraction pattern of the new polymorphic form was recorded with a Bruker D2-Phaser diffractometer with the following parameters:
- Tube anode: Cu
- Generator tension (kV): 30
- Generator current (mA): 10
- Wavelengths α1 and α2 (Å): 1.54056, 1.54439
- Intensity ratio (α2/α1): 0.500
- Spinner: off
- Angular range (2θ°): 2.00 - 50.00
- Step size (2θ°): 0.020
- Time per step (sec): 3.0

DSC analysis was performed using a Mettler DSC1 System. Heat flow was recorded from 30 to 250°C with linear heating rate (10°C/min), using closed aluminum crucibles (40 µl volume) with a pinhole, under a 50 ml/min nitrogen flow. About 5 mg of powder were used for each measurement. The thermal profile was acquired and elaborated by Mettler software STARe Thermal Analysis System.

The infrared spectrum was recorded in Attenuated Total Reflectance (ATR) mode using Fourier-Transform spectrometer Nicolet iS10 Thermo Scientific, equipped with Specac ATR Golden Gate and OMNIC software (Thermo Scientific). The spectrum was the result of the acquisition and transformation of 32 co-added scans in the 4000-550 cm⁻¹ spectral region at a resolution of 4 cm⁻¹.

The XRPD spectrum is shown in figure 1 and is characterized by main peaks at 2θ angle: 5.7 - 6.3 - 8.0 - 11.7 - 12.7 - 15.3 - 16.0 - 17.0 - 19.1 - 19.6 - 20.2 - 20.4 - 23.2 - 24.1 - 25.1 - 25.5 - 26.3 - 27.5 - 29.1.

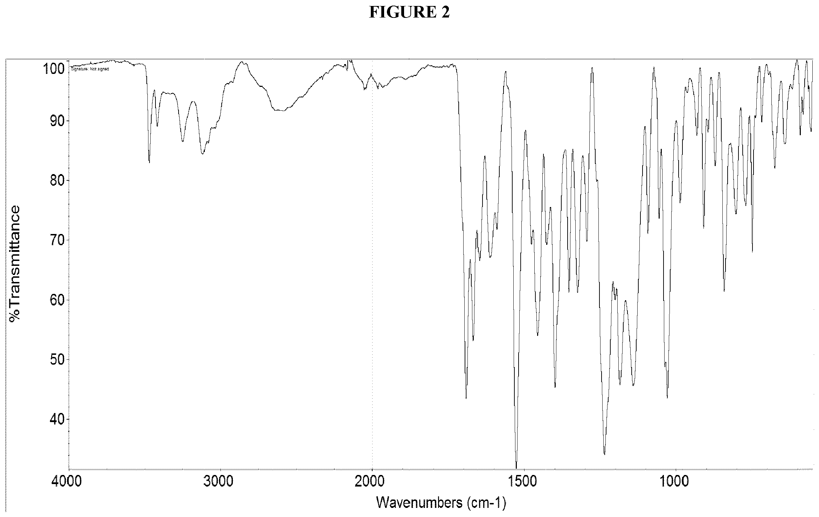

The FTIR spectrum, shown in Figure 2, has the following intense absorption bands: 3468 - 3415 - 3247 - 3113 - 1690 - 1667 - 1525 - 1455 - 1398 - 1353 - 1324 - 1234 - 1185 - 1141 - 1028 - 840 - 749 ± 2 cm⁻¹.

The DSC profile is characterized by a broad endothermic signal below 100°C due to release of residual solvent/moisture, and a melting peak with onset at about 180°C, maximum at about 187°C and ΔH of about - 46 J/g. Decomposition takes place upon melting, at about 190-200°C.

## Claims

1. A polymorphic form of Lenvatinib mesylate **characterized by** XRPD spectrum showing the main peaks at 2θ angle: 5.7 - 6.3 - 8.0 - 11.7 - 12.7 - 15.3 - 16.0 - 17.0 - 19.1 - 19.6 - 20.2 - 20.4 - 23.2 - 24.1 - 25.1 - 25.5 - 26.3 - 27.5 - 29.1 at the Cu α1 and α2 wavelengths.

2. A pharmaceutical composition comprising the crystalline form of claim 1 as active ingredient.

3. A process for the preparation of the polymorphic form of claim 1 comprising micronization of Lenvatinib mesylate, ACA-1-HT dry form.
